(19) Europäisches Patentamt
European Patent Office
Office européen des brevets

(11) **EP 2 233 158 A2**

(12) **EUROPEAN PATENT APPLICATION**

(43) Date of publication:
**29.09.2010 Bulletin 2010/39**

(51) Int Cl.:
***A61L 15/30*** *(2006.01)*

(21) Application number: **10250565.8**

(22) Date of filing: **25.03.2010**

| | |
|---|---|
| (84) Designated Contracting States:<br>**AT BE BG CH CY CZ DE DK EE ES FI FR GB GR HR HU IE IS IT LI LT LU LV MC MK MT NL NO PL PT RO SE SI SK SM TR**<br>Designated Extension States:<br>**AL BA ME RS** | • **Kasahara, Tsuyoshi**<br>  **Ibaraki-shi, Osaka 567-8680 (JP)**<br>• **Hamada, Atsushi**<br>  **Ibaraki-shi, Osaka 567-8680 (JP)**<br>• **Ishikura, Jun**<br>  **Ibaraki-shi, Osaka 567-8680 (JP)** |
| (30) Priority: **25.03.2009 JP 2009073408** | (74) Representative: **Duckworth, Timothy John**<br>**J.A. Kemp & Co.**<br>**14 South Square**<br>**Gray's Inn**<br>**London WC1R 5JJ (GB)** |
| (71) Applicant: **Nitto Denko Corporation**<br>**Ibaraki-shi, Osaka 567-8680 (JP)** | |
| (72) Inventors:<br>• **Funakoshi, Yoshio**<br>  **Ibaraki-shi, Osaka 567-8680 (JP)** | |

(54) **Patch and patch preparation**

(57)    The present invention provides a patch and a patch preparation having an adhesive layer with a sufficient thickness, as well as appropriate skin adhesiveness and flexibility, which resist falling off from the skin even when a load is applied to the side opposite to the surface of the patch or patch preparation to be adhered to the skin, i.e., an exposed surface of a support of the patch or patch preparation, and show low irritation to the skin; an adhesive layer, which is obtained by crosslinking a liquid rubber having a crosslinkable functional group in a molecule in the presence of a crosslinking agent having a functional group number of 3 or below and a catalyst, on at least one surface of a support; and a patch preparation having an adhesive layer containing a pharmaceutical agent.

**Description**

TECHNICAL FIELD OF THE INVENTION

The present invention relates to a patch and a patch preparation showing appropriate skin adhesiveness and flexibility, which resist falling off from the skin even when a load is applied to the exposed surface of a support of the patch or patch preparation, show low irritation to the skin, and can be preferably used for the protection of a skin lesion or a wounded surface, transdermal absorption of a drug and the like.

BACKGROUND OF THE INVENTION

Conventionally, patch and patch preparation for the skin and the like have been used for the protection of the skin lesion and a wounded surface, transdermal absorption of a drug and the like. Such patch and patch preparation generally have a constitution wherein a layer made of an adhesive composition containing a drug as necessary, namely, an adhesive layer, is formed on at least one surface of a flexible support. The aforementioned adhesive layer is required to show skin adhesiveness. This is because a patch or patch preparation needs to maintain an adhesion state without being detached from the skin surface for a given period. On the other hand, when skin adhesiveness of a patch or patch preparation is too strong, irritation to the skin becomes higher during detachment thereof from the skin, thus rendering the patch or patch preparation unsuitable for adhesion to the skin. In other words, a patch or patch preparation is also required to show low irritation to the skin. Consequently, a patch or patch preparation is required to show conflicting adhesive properties. As a technique to solve the conflicting adhesive properties required of a patch or patch preparation, use of an acrylic gel composition as an adhesive layer has been proposed.

For example, an acrylic gel composition wherein an organic liquid component is held by crosslinked acrylic adhesive is disclosed (patent document 1). However, since an adhesive layer having a certain level of thickness is somewhat difficult to form on a support using the aforementioned acrylic gel composition, the layer has a limited ability to retain or absorb a drug, an organic liquid component such as a transdermal absorption promoter and the like, and the like, and therefore, cannot hold them in large amounts. Since an adhesive layer having a large thickness is difficult to form using the above-mentioned compositions, when a patch and patch preparation containing the above-mentioned compositions as an adhesive layer is adhered to the skin, an improvement in the ability to absorb external impacts and the like to protect the skin is necessary.

When a patch or patch preparation is adhered to the skin, a load is applied to the surface opposite to the adhesive layer, i.e., exposed surface of a support, due to clothes, sheet and the like, and the patch or patch preparation may be detached. However, not to mention a technique capable of solving the problem, even such problem has not been raised in the above-mentioned document. In addition, the above-mentioned document does not disclose or suggest a crosslinking treatment of an adhesive layer in the presence of a catalyst and using a crosslinking agent.

PRIOR ART DOCUMENT

PATENT DOCUMENT

patent document 1: JP-A-3-220120

SUMMARY OF THE INVENTION

PROBLEMS TO BE SOLVED BY THE INVENTION

Accordingly, the present invention aims to provide a patch and a patch preparation having an adhesive layer with a sufficient thickness, as well as appropriate skin adhesiveness and flexibility, which resist falling off from the skin even when a load is applied to the surface opposite to the surface of the patch or patch preparation to be adhered to the skin, i.e., an exposed surface of a support of the patch or patch preparation, and show low irritation to the skin.

MEANS OF SOLVING THE PROBLEMS

The present inventors have conducted intensive studies in an attempt to solve the aforementioned problems and found that the above-mentioned problems can be solved by forming an adhesive layer, which comprises a liquid rubber having a crosslinkable functional group in a molecule thereof in the presence of a crosslinking agent having a functional group number of 3 or below and a catalyst, on at least one surface of a support, which resulted in the completion of the present invention.

Accordingly, the present invention relates to the following [1] - [10].

[1] A patch comprising a support and an adhesive layer formed on at least one surface of the support, wherein the adhesive layer comprises a liquid rubber having a crosslinkable functional group in a molecule thereof and is crosslinked in the presence of a crosslinking agent having a functional group number of 3 or below and a catalyst.

[2] The patch of the above-mentioned [1], wherein the liquid rubber is a liquid isoprene rubber having a crosslinkable functional group in a molecule.

[3] The patch of the above-mentioned [1] or [2], wherein the crosslinking is performed under conditions such that the proportion of the total number of the functional groups of the crosslinking agent to the total number of the functional groups of the liquid rubber is 30% - 100%.

[4] The patch of any of the above-mentioned [1] to [3],
wherein the adhesive layer is crosslinked in the presence of an organic liquid component.

[5] The patch of the above-mentioned [4], wherein the organic liquid component is one or more kinds selected from a fat, an oil, a hydrocarbon, a fatty acid ester and a higher fatty acid.

[6] The patch of the above-mentioned [5], wherein the fatty acid ester is a fatty acid monoalkyl ester.

[7] The patch of the above-mentioned [6], wherein the fatty acid monoalkyl ester is one or two kinds selected from isopropyl myristate and isopropyl palmitate.

[8] The patch of any of the above-mentioned [1] to [7],
wherein the adhesive layer is crosslinked in the presence of a tackifier.

[9] The patch of any one of the above-mentioned [1] to [8],
wherein the adhesive layer has a thickness of not less than 40 $\mu$m.

[10] A patch preparation comprising the patch of any one of the above-mentioned [1] to [9], wherein the adhesive layer comprises a drug.

EFFECT OF THE INVENTION

In the present invention, since use of a crosslinking agent having a functional group number of 3 or below enables suppression of an excessive increase in the crosslinking density of the adhesive layer, even when a load by clothes, sheet and the like is applied to the surface opposite to the adhesion surface of a patch or patch preparation to the skin, i.e., an exposed surface of a support, the patch or patch preparation less apt to easily fall off from the skin. In addition, a crosslinking reaction in the presence of a catalyst, enables to suppress an excessive decrease in the crosslinking density of the adhesive layer even when a crosslinking agent having a functional group number of 3 or below is used, and therefore, a decrease in the intensity of the adhesive layer can be suppressed. As a result, a patch and a patch preparation less irritative to the skin and less apt to easily fall off from the skin even when a load is applied to an exposed surface of a support of the patch or patch preparation can be obtained.

MODE FOR CARRYING OUT THE INVENTION

In the present invention, the above-mentioned liquid rubber having a crosslinkable functional group in a molecule forms a net-like structure upon crosslinking of the composition containing the liquid rubber, and confers flexibility and shape retainability to the adhesive layer. The liquid rubber is not particularly limited as long as it has a crosslinkable functional group in a molecule, and liquid isoprene rubber containing the aforementioned functional group, liquid butadiene rubber containing the aforementioned functional group, liquid isobutylene rubber containing the aforementioned functional group and the like can be used. One or more kinds of liquid rubbers can be used. Such liquid rubber is liquid even though it has a high molecular weight, and enables an adhesive layer to contain a large amount of organic liquid component with ease. The aforementioned liquid isoprene rubber containing a functional group is preferably used, since it can easily confer flexibility to an adhesive layer. In the present specification, the "liquid" means flowability at 25°C. The presence or absence of flowability is evaluated by filling a sample to a half height of a bottle (inner diameter 50 mm, height 100 mm), and observing the deformation when the bottle is tilted by 90 degrees.

The weight average molecular weight of a liquid rubber having a crosslinkable functional group in a molecule to be used in the present invention is not particularly limited as long as the rubber component is liquid. It is preferably 1,000 - 60,000, more preferably 10,000 - 40,000, most preferably 20,000 - 30,000. When the weight average molecular weight is less than 1,000, the flexibility of the adhesive layer may decrease, and a large amount of an organic liquid component may not be easily contained in an adhesive layer. On the other hand, when the weight average molecular weight exceeds 60,000, the rubber may not be liquid, the content uniformity of the adhesive layer may be difficult to maintain, and the compatibility of the aforementioned liquid rubber with other components contained in an adhesive layer may be difficult to secure.

Here, the "weight average molecular weight" is measured by gel filtration chromatography under the following conditions.

(analysis conditions)

Gel filtration chromatography apparatus: HLC8120 (manufactured by Tosoh Corporation) column: TSK gel GMH-H(S) (manufactured by Tosoh Corporation) standard: polystyrene eluent: tetrahydrofuran
flow rate: 0.5 mL/min
measurement temperature: 40°C
detector: differential refractometer

In the patch and patch preparation of the present invention, the composition used for the preparation of the adhesive layer contains a liquid rubber having a crosslinkable functional group in a molecule in an proportion of preferably 25 wt% - 65 wt%, more preferably 30 wt% - 60 wt%, of the total weight of the composition. When the amount of the aforementioned liquid rubber is lower than 25 wt%, the adhesive layer formed may fail to achieve a sufficient crosslinking density, and formation of an adhesive layer may become difficult. On the other hand, when the amount of the aforementioned liquid rubber exceeds 65 wt%, the adhesive layer thus formed may have decreased flexibility and may fall off from the skin.

While the crosslinkable functional group in the above-mentioned liquid rubber is not particularly limited, amino group, carboxyl group, hydroxyl group, chlorosulfone group, acyloxy group, epoxy group, isocyanate group, methylol group, sulfone group, mercapto group and the like may be used. The functional group may be used alone or in a combination of two or more kinds thereof. In consideration of formation of crosslinking with an epoxy compound as the below-mentioned crosslinking agent, carboxyl group and hydroxyl group are preferable.

While the number of crosslinkable functional groups in a molecule of the above-mentioned liquid rubber is not particularly limited, when not less than three functional groups are present in a molecule, the aforementioned liquid rubber generally forms a net-like structure efficiently, and the effect of the invention can be sufficiently afforded. In view of the above, the number of crosslinkable functional groups in a molecule of the aforementioned liquid rubber is generally not less than 3, preferably not less than 5, more preferably not less than 8, further more preferably not less than 10.

In the present invention, since the above-mentioned liquid rubber has not less than 3 crosslinkable functional groups in a molecule, a three-dimensional net-like structure can be formed even without introducing a branching agent and the like into the main chain of the liquid rubber. Therefore, a large amount of an organic liquid component can be contained in the adhesive layer.

On the other hand, when the number of the aforementioned functional groups in a molecule is too many, the amount of the necessary crosslinking agent increases. Thus, the number of the crosslinkable functional groups in a molecule is preferably not more than 20, more preferably not more than 15, further preferably not more than 12. When the number of the functional groups in a molecule is too many, the remaining active groups increase in number, possibly affecting the components contained in an adhesive layer such as drugs and the like, and the flexibility of an adhesive layer may decrease, which may cause the patch falling off from the skin or result in poor followability of the patch to the skin deformation.

The number of crosslinkable functional groups in a molecule of liquid rubber is measured as follows. That is, the total number of crosslinkable functional groups in a molecule of liquid rubber in a sample is determined, and the number is divided by the number average molecular weight of the liquid rubber to give the number of crosslinkable functional groups per one molecule of liquid rubber. The number of such functional groups can be determined according to the method described in the Japanese Industrial Standards (JIS) K0070, and the like. For example, when the functional group is a carboxyl group, the number of carboxyl groups in a sample is determined by an acid number measurement method using potassium hydroxide. When it is an acyloxy group, the number can be determined by an ester number measurement method using potassium hydroxide, and when it is a hydroxyl group, the number can be determined by a hydroxyl group number measurement method including acetylation of a sample and neutralization thereof with potassium hydroxide. Here, the number average molecular weight of liquid rubber means a value obtained under the same conditions as the aforementioned weight average molecular weight.

In the present invention, an adhesive layer formed on at least one surface of a support has a gel fraction of preferably 45 wt% - 85 wt%, more preferably 45 wt% - 70 wt%. When the gel fraction is lower than 45 wt%, the adhesive layer formed does not have a sufficient crosslinking density, and formation of an adhesive layer may become difficult. On the other hand, when the gel fraction exceeds 85 wt%, the adhesive layer formed may have decreased flexibility and may fall off from the skin.

In the present invention the "gel fraction" means the ratio of the weight of insoluble matter obtained by immersing an adhesive layer in toluene, to the components involved in the crosslinking of the adhesive layer. The gel fraction can be determined by immersing a sample of the composition for forming an adhesive layer in toluene at ambient temperature (23°C) for 7 days and applying the weight of the obtained insoluble matter to the following equation.

$$gel\ fraction\ (wt\%) = W_2/(W_1 \times A/B) \times 100$$

A: weight of liquid rubber having crosslinkable functional group in a molecule and crosslinking agent
B: total weight of adhesive layer
$W_1$: weight of sample taken from adhesive layer
$W_2$: weight of insoluble matter obtained by immersing, in toluene, sample taken from adhesive layer

The above-mentioned gel fraction in the present invention can be achieved by applying an adhesive layer to a crosslinking treatment. In the present invention, a crosslinking treatment is performed using a crosslinking agent having a functional group number of 3 or below and in the presence of a catalyst. As a result, an excessive increase and decrease in the crosslinking density of the adhesive layer can be suppressed, and appropriate intensity, appropriate flexibility and appropriate adhesiveness can be imparted to the adhesive layer. In addition, even when a load is applied to the surface opposite to the surface of a patch or patch preparation to be adhered to the skin, i.e., an exposed surface of a support of the patch or patch preparation, it resists falling off from the skin and is less irritant to the skin.

Examples of the crosslinking agent having a functional group number of 3 or below to be used in the present invention include amine compounds such as diaminohexane and the like, bifunctional epoxy compounds such as polyethylene glycol diglycidyl ether, polypropylene glycol diglycidyl ether and the like, bifunctional or trifunctional epoxy compounds such as trimethylolpropane polyglycidyl ether and the like, isocyanate compounds such as aromatic diisocyanates (e.g., trilene diisocyanate, diphenylmethane diisocyanate, xylylene diisocyanate and the like), aliphatic diisocyanates (e.g., hexamethylene diisocyanate and the like), alicyclic diisocyanates (e.g., isophorone diisocyanate, hydrogenated diphenylmethane diisocyanate and the like), and the like, organic peroxides such as peroxyketal, dialkyl peroxide, diacyl peroxide and the like, organic metal salts such as acetylacetone aluminum salt, acetylacetone vanadium salt, acetylacetone molybdenum salt and the like, metal alcoholates such as tetrabutyl titanate and the like, metal chelate compounds such as aluminum glycinate, aluminum hydroxide gel and the like, and the like. In the present invention, one kind therefrom may be used alone or in a combination of two or more kinds thereof. When the crosslinkable functional group in a molecule of liquid rubber is a carboxyl group, an epoxy compound is preferably used among the aforementioned crosslinking agents. This is because an epoxy group reacts with a carboxyl group in a molecule of liquid rubber and develops a hydroxyl group, and the hydroxyl group can react with other carboxyl group, whereby a net-like structure can be advantageously formed.

The amount of the above-mentioned crosslinking agent needs to be such an amount as allows formation of a sufficient crosslinking structure in an adhesive layer. Specifically, it is determined by the total number of the aforementioned functional groups of a liquid rubber having a crosslinkable functional group in a molecule, and the total number of the functional groups of a crosslinking agent to be added, which are contained in a composition to be used for the preparation of an adhesive layer. Preferably, it is 0.01 wt% - 4 wt%, more preferably 0.05 wt% - 2 wt%, of the total amount of the composition to be used for preparation of the adhesive layer.

As the catalyst, tertiary amine catalyst, organic metal catalyst, and various other catalysts can be used. In view of reactivity, organic metal catalysts are preferable. While the organic metal catalyst is not particularly limited, di-n-butyl tin dilaurate, tri-n-butyl tin chrolide, tetra n-butyl tin, o-phenylsodium, potassium oleate, 2-ethylhexyl titanate, iron 2-ethylhexoate, manganese naphthenate, zinc naphthenate, copper naphthenate, lead naphthenate, nickel naphthenate, cobalt naphthenate and the like can be mentioned. Metal naphthenate catalysts, such as manganese naphthenate, zinc naphthenate, copper naphthenate, nickel naphthenate, cobalt naphthenate and the like are more preferably used in view of reaction rate and safety.

The amount of the above-mentioned catalyst to be added is not particularly limited as long as it can appropriately promote a crosslinking reaction. It is appropriately about 0.01 wt% - 2.00 wt% of the total amount of a composition to be used for the preparation of an adhesive layer.

In the present invention, moreover, in a composition to be used for the preparation of an adhesive layer, the proportion of the total number of the functional groups of a crosslinking agent to the total number of the aforementioned functional groups in a liquid rubber having a crosslinkable functional group in a molecule (hereinafter to be referred to as "functional group ratio") is preferably 30% - 100%. Setting the functional group ratio to fall within the aforementioned range, an adhesive layer with a sufficient thickness can be formed and suitable intensity, flexibility and adhesiveness can be imparted to an adhesive layer.

In the patch and patch preparation of the present invention, the adhesive layer can contain an organic liquid component. The organic liquid component is combined with a net-like structure formed by crosslinking of the above-mentioned liquid rubber, and enhances the flexibility of the adhesive layer. When a chemical substance such as drugs and the like is contained in the adhesive layer, the organic liquid component dissolves or disperses the substance. The organic liquid component to be used in the present invention needs to have compatibility with the above-mentioned liquid rubber in the adhesive layer. An organic liquid component which is liquid and nonvolatile at ambient temperature is preferable. In the present invention, a fat or an oil such as olive oil, castor oil, lanolin and the like, a hydrocarbon such as squalane and liquid paraffin, a fatty acid ester such as fatty acid alkyl ester and the like, a higher fatty acid such as oleic acid, caprylic acid and the like are preferable. The organic liquid component may be used alone, or in a mixture of two or more kinds thereof.

Particularly, as in the patch preparation of the present invention, when a drug is contained in an adhesive layer, a fatty acid ester is preferably used to promote transdermal absorption of the drug. Examples of the fatty acid ester include a fatty acid dialkyl ester such as dioctyl phthalate, diisopropyl adipate, diethyl sebacate and the like, a fatty acid monoalkyl ester and the like. To enhance good transdermal absorption of a drug, a fatty acid monoalkyl ester is preferable.

When the carbon number of a fatty acid constituting a fatty acid monoalkyl ester is greater or smaller than necessary, compatibility with the above-mentioned liquid rubber may be degraded or a fatty acid monoalkyl ester may be volatilized in the heating step during formulation of a preparation.

In the present invention, therefore, a fatty acid monoalkyl ester comprised of a saturated or unsaturated higher fatty acid having a carbon number of 12 - 16, more preferably 12 - 14, and a saturated or unsaturated lower monovalent alcohol having a carbon number of 1 - 4 is preferably employed. For preservation stability, a fatty acid monoalkyl ester comprised of a saturated higher fatty acid and a saturated lower monovalent alcohol is preferably used since it is free of oxidative decomposition and the like. Examples of the aforementioned higher fatty acid include lauric acid (C12), myristic acid (C14) and palmitic acid (C16), and myristic acid and palmitic acid are particularly preferable. Examples of the aforementioned lower monovalent alcohol include methyl alcohol, ethyl alcohol, propyl alcohol and butyl alcohol. They are not limited to a straight chain alcohol and may be a branched chain alcohol. Particularly preferred is isopropyl alcohol. Most preferable fatty acid monoalkyl esters usable in the present invention are isopropyl myristate and isopropyl palmitate.

The aforementioned organic liquid component is preferably contained in a proportion of 10 wt% - 40 wt%, more preferably 15 wt% - 30 wt%, of the total amount of the composition to be used for preparation of an adhesive layer. When the content of the organic liquid component is less than 10 wt% of the total amount of the composition, the adhesive layer obtained after crosslinking reaction becomes too hard, thus possibly degrading the adhesiveness of the adhesive layer to the skin or followability to the skin. In addition, diffusional mobility and absorption of other components such as drugs etc. in the adhesive layer may be degraded. On the other hand, when the content of the organic liquid component exceeds 40 wt% of the total amount of the composition, the strength of the adhesive layer may decrease to cause adhesive residue.

The patch and patch preparation of the present invention may further contain a general tackifier during preparation of an adhesive layer. Examples of the tackifier include naturally occurring substance and a derivative thereof such as rosin resin, terpene resin and the like, synthetic resins such as aliphatic petroleum resin, alicyclic petroleum resins such as alicyclic saturated hydrocarbon resin, petroleum resin, aromatic petroleum resin, coumarone-indene resin, styrene resin, phenol resin, xylene resin and the like, and the like. These tackifiers are preferably contained in a proportion of 15 wt% - 60 wt%, further preferably 30 wt% - 50 wt%, of the total amount of the composition to be used for preparation of an adhesive layer. When it is less than 15 wt%, the adhesive layer may not have sufficient adhesiveness, and when it is greater than 60 wt%, the tackifier may be separated in the adhesive layer to cause stickiness of the adhesive layer and leave adhesive residue when the adhesive layer is removed from the skin.

In the patch and patch preparation of the present invention, the adhesive layer may contain other optional components within the range that does not prevent the effect of the invention. For example, antioxidants such as ascorbic acid, tocopherol acetate, natural vitamin E, dibutylhydroxytoluene, butylhydroxyanisole and the like, amine-ketone anti-aging agents such as 2,6-tert-butyl-4-methylphenol and the like, aromatic secondary amine anti-aging agents such as N,N'-di-2-naphthyl-p-phenylenediamine and the like, monophenol anti-aging agents such as 2,2,4-trimethyl-1,2-dihydroquinoline polymer and the like, bisphenol anti-aging agents such as 2,2'-methylene-bis(4-ethyl-6-tertbutylphenol) and the like, polyphenol anti-aging agents such as 2,5-tert-butylhydroquinone and the like, fillers such as kaolin, hydrated silicon dioxide, zinc oxide, acrylic acid starch 1000 and the like, softening agents such as propylene glycol, polybutene, macrogol 1500 and the like, preservatives such as benzoic acid, sodium benzoate, chlorhexidine hydrochloride, sorbic acid, methyl p-hydroxybenzoate, butyl p-hydroxybenzoate and the like, colorants such as yellow iron oxide, yellow iron sesquioxide, iron sesquioxide, black iron oxide, carbon black, carmine, β-carotene, copper chlorophyll, Food Color blue No. 1, Food Color yellow No. 4, Food Color Red No. 2, licorice extract and the like, algefacients such as fennel oil, d-camphor, dl-camphor, peppermint oil, d-borneol, 1-menthol and the like, flavors such as spearmint oil, clove oil, vanillin, bergamot oil, lavender oil and the like, and the like can be contained.

The patch and patch preparation of the present invention can be produced generally as follows. That is, the above-mentioned liquid rubber and an organic liquid component are mixed in the presence of a solvent where necessary, a crosslinking agent and catalyst are added thereto, the viscosity is adjusted where necessary with an organic solvent, and the mixture is applied to at least one surface of a support. Then, the solvent is evaporated at a suitable temperature to form crosslinking in the liquid rubber, thereby providing an adhesive layer on the support. Where necessary, the adhesive layer is aged. Furthermore, a release liner described below can also be press-bonded to give a laminate. In addition, an adhesive layer may be formed on the release liner, and a support may be press-bonded to an adhesive face of the adhesive layer.

While the support to be used in the present invention is not particularly limited, a support that does not substantially allow permeation of components contained in the adhesive layer, namely, a support that does not allow components contained in the adhesive layer to pass through the support and get lost from the back face of the support is preferable. As the above-mentioned support, a flexible material is preferably used since it is applied as a patch to the skin. Examples of the material include a film or sheet made of a synthetic resin such as plasticized flexible polyvinyl chloride, nonplasticized flexible polyvinyl chloride, polyethylene, polypropylene, polybutene, ethylene-methylmethacrylate copolymer, ethylene-vinyl acetate copolymer, ethylene-propylene rubber, polyamide, poly(ethylene terephthalate), polyurethane, polyvinyldene chloride, polyvinyl alcohol and the like. In addition, flexible films and sheets of non-woven fabric, foamed plastic sheet, cellulose, acetyl cellulose and the like can also be used. Moreover, a release liner made of such materials can also be used.

Examples of the release liner usable in the present invention include glassine paper, polyethylene, polypropylene, polyester, poly(ethylene terephthalate), polystyrene, aluminum film, foamed polyethylene film, foamed polypropylene film etc., or a laminate of members selected therefrom, or those mentioned above with silicone processing, emboss processing etc., and the like.

In the patch and patch preparation of the present invention, an adhesive layer having a certain level of thickness can be formed on a support. As a result, an effect of protecting the skin lesion and a wounded surface from impacts on the skin and the like can be improved, and the adhesive layer can contain a large amount of a drug and the like. Accordingly, in the patch and patch preparation of the present invention, the adhesive layer preferably has a thickness of not less than 40 $\mu$m, more preferably not less than 100 $\mu$m. On the other hand, the thickness of the adhesive layer should be up to around 400 $\mu$m, preferably 300 $\mu$m. When the thickness exceeds 400 $\mu$m, an applied patch or patch preparation may be misaligned to allow protrusion of an adhesive component, or adhesive residue on the skin.

The patch of the present invention can be provided as a medical material or hygienic material in the form of a sheet-like patch, film-like patch, pad-like patch and the like, and can be used as an alternative of gauze in adhesive plaster, an alternative of non-woven fabric in wound-covering dressings and the like for the protection of a lesion and a wounded part of the skin. In addition, the patch preparation of the present invention contains a drug in an adhesive layer formed on a support, and can be provided as a matrix-type patch preparation, a reservoir-type patch preparation, a patch-type transdermal preparation and the like.

The drug to be used for the patch preparation of the present invention is preferably one that can be transdermally administered to mammals such as human and the like. Specific examples of the drug include general anesthetic, topical anesthetic, antipsychotic agents, antidepressant, mood-stabilizing drug, psychostimulant drug, sleep inducing drug, antianxiety drug, antiepileptic drug, therapeutic drug for Parkinson's disease, migraine-abortive agent, cerebral circulation and metabolism improver, antidementia drug, narcotic, anti-vertiginous drug, autonomic drug, antispasmodic drug, muscle relaxant, anti-histamine drug, cardiac stimulant, antiarrhythmic, diuretic, hypotensive drug, vasoconstrictor, coronary vasodilator, peripheral vasodilator, hyperlipidemia drug, circulatory organ drug, anapnoic, antitussive expectorant, drug for peptic ulcera therapeutic, hormone drug, antipyretic analgesic antiphlogistic drug, antipruritic drug, astringent drug, hemostatic, gout treatment drug, diabetes drug, anti-malignant tumor drug, antibiotic, chemical therapy drug, antiparasitic drug, quit smoking aid and the like can be mentioned. The content of the aforementioned drug(s) in the adhesive layer is not particularly limited as long as it is an effective amount capable of exhibiting efficacy upon transdermal absorption and the properties of the adhesive layer of the patch preparation of the present invention are not impaired, and is preferably 0.01 wt% - 60 wt%.

EXAMPLES

The present invention is explained in more detail in the following by referring to Examples.

[Examples 1 - 7 and Comparative Examples 1 - 3]

The formulations of the patches of Example 1 - 7 of the present invention, and the patches of Comparative Examples 1 - 3 are shown in Table 1. In each patch of the Examples and Comparative Examples, carboxylated liquid polyisoprene (weight average molecular weight=25,000, number of carboxyl groups in a molecule=10) as a liquid rubber having a crosslinkable functional group in a molecule, isopropyl myristate as an organic liquid component, and an alicyclic hydrocarbon resin (softening point=100˚C) as a tackifier were used. As the crosslinking agent, those shown below were used, and as the catalyst, zinc naphthenate was used.

<Crosslinking agent>

Examples 1 - 7 and Comparative Example 3: trimethylolpropane polyglycidyl ether (trifunctional)

Comparative Examples 1 and 2: 1,3-bis(N,N-diglycidyl aminomethyl)cyclohexane (tetrafunctional)

The patches of the Examples and Comparative Examples were prepared as follows. First, a liquid rubber having a crosslinkable functional group in a molecule, an organic liquid component and a tackifier were mixed in the presence of an organic solvent, and then a crosslinking agent and a catalyst were added to give a liquid for forming an adhesive layer. Table 1 shows the amounts of components other than the organic solvent. The liquid was applied to a release liner of (silicone-treated poly(ethylene terephthalate) such that the thickness after drying was 200 $\mu$m for Examples other than Example 4, and 100 $\mu$m for Example 4, and the organic solvent was removed by drying. Then, a support of poly(ethylene terephthalate) was press-bonded to an adhesive face of the adhesive layer and the laminate was aged at 60˚C for 3 days to give a patch. As the organic solvent, toluene was used, and the thickness of the adhesive layer of each test piece (width 30 mmxlength 50 mm) of the Examples and Comparative Examples was measured using a dial gauge defined in the Japanese Industrial Standards (JIS) B7503.
Table 1

| | amount added (parts by weight) | | | | |
|---|---|---|---|---|---|
| | liquid rubber | tackifier | organic liquid component | crosslinking agent | catalyst |
| Example 1 | 100 | 100 | 50 | 5.80 | 0.30 |
| Example 2 | 100 | 100 | 50 | 4.64 | 0.30 |
| Example 3 | 100 | 100 | 50 | 4.06 | 0.30 |
| Example 4 | 100 | 100 | 50 | 3.48 | 0.30 |
| Example 5 | 100 | 100 | 50 | 2.90 | 0.30 |
| Example 6 | 100 | 100 | 50 | 2.32 | 0.30 |
| Example 7 | 100 | 100 | 50 | 1.74 | 0.30 |
| Comparative Example 1 | 100 | 100 | 50 | 1.10 | 0 |
| Comparative Example 2 | 100 | 100 | 50 | 1.10 | 0.30 |
| Comparative Example 3 | 100 | 100 | 50 | 1.74 | 0 |

The content (wt%) of each component to the total weight of the adhesive layer, the kind of the crosslinking agent used and the functional group ratio are shown in Table 2.
Table 2

| | component | | | | | | functional group ratio[*3] |
|---|---|---|---|---|---|---|---|
| | liquid rubber | tackifier | organic liquid compo-nent | crosslinking agent | | catalyst | |
| | (wt%) | (wt%) | (wt%) | (wt%) | kind | (wt%) | (%) |
| Example 1 | 39.05 | 39.05 | 19.52 | 2.26 | A[*1] | 0.12 | 100 |
| Example 2 | 39.22 | 39.22 | 19.61 | 1.82 | A | 0.12 | 80 |
| Example 3 | 39.31 | 39.31 | 19.66 | 1.60 | A | 0.12 | 70 |
| Example 4 | 39.40 | 39.40 | 19.70 | 1.37 | A | 0.12 | 60 |
| Example 5 | 39.49 | 39.49 | 19.75 | 1.15 | A | 0.12 | 50 |
| Example 6 | 39.59 | 39.59 | 19.79 | 0.92 | A | 0.12 | 40 |

(continued)

| | component | | | | | | |
|---|---|---|---|---|---|---|---|
| | liquid rubber | tackifier | organic liquid compo-nent | crosslinking agent | | catalyst | functional group ratio*3 |
| | (wt%) | (wt%) | (wt%) | (wt%) | kind | (wt%) | (%) |
| Example 7 | 39.68 | 39.68 | 19.84 | 0.69 | A | 0.12 | 30 |
| Comparative Example 1 | 39.83 | 39.83 | 19.91 | 0.44 | B*2 | 0 | 30 |
| Comparative Example 2 | 39.78 | 39.78 | 19.89 | 0.44 | B | 0.12 | 30 |
| Comparative Example 3 | 39.72 | 39.72 | 19.86 | 0.69 | A | 0 | 30 |

*1; trimethylolpropane polyglycidyl ether
*2; 1,3-bis(N,N-diglycidyl aminomethyl)cyclohexane
*3; proportion (%) of total number of functional groups to be added to total number of functional groups of liquid rubber component to be added

Each patch of the above-mentioned Examples and Comparative Examples was evaluated for (1) shape retention; (2) constant load (anchorage) and (3) ball tackiness value as indices of easiness of falling of patch; and (4) skin irritation. Each evaluation method is as follows.

(1) Shape retention

The prepared patches were observed for the level of crosslinking formation and the presence or absence of shape retention, and evaluated as follows.

○; crosslinking is sufficiently formed, and shape retention is shown
×; crosslinking is not sufficiently formed and shape retention is not shown

(2) Constant load (anchorage)

The constant load (anchorage) was measured by the following method as an index showing anchorage of a patch to the skin surface when an external load is applied to the exposed surface of a support, namely, resistance to falling off from the skin. First, each patch of the Examples and Comparative Examples was punched out into a 12 mmx80 mm size to give a test piece. For the measurement, a steel rule (manufactured by SHINWA) was used to measure the distance of detachment of the test piece, and a stop watch (manufactured by SEIKO) was used to measure the detachment time. A test piece prepared from each patch was adhered to an adherend (collagen plate), and a load (15 g) was hung on the other end in the form of a 90° detachment test. The detachment rate was calculated from the distance and time of peeling of the test piece, and evaluated according to the following evaluation criteria. The detachment failure mode is also shown below.

<Failure mode>

K (interface failure): adhesive does not remain on adherend upon peeling off of adhesive tape from the adherend
T (anchor failure): adhesive is released from substrate and adhesive remains on adherend upon peeling off of adhesive tape from the adherend

<Evaluation criteria>

◎; less than 200 mm/h
○; not less than 200 mm/h less than 300 mm/h
×; not less than 300 mm/h

(3) Ball tackiness value

The ball tackiness value was measured according to Japanese Industrial Standards (JIS) Z0237 as an index showing easiness of falling off of a patch from the skin when an external load is applied to the exposed surface of a support upon adhesion of the patch to the skin. In this test, each patch of the Examples and Comparative Examples was punched out into width 50 mm×length 50 mm and used as a test piece for convenience. To be precise, under the conditions of 23°C, relative humidity 65%, the test piece was set on a ball rolling apparatus at an inclination angle of 30° in such a manner that the adhesive face was exposed and fixed to avoid difference in the level of surface between the test piece and the approach. Then, balls having different diameters were rolled on the test piece, and the diameter of the largest ball (ball No.) that stopped still thereon for 5 seconds or longer was taken as a ball tackiness of the test piece, and evaluated according to the following evaluation criteria.

<Evaluation criteria>

⊙; not less than 17
○; not less than 16 and less than 17
×; less than 16

(4) Skin irritation

Each patch of the Examples and Comparative Examples was adhered to the back of a human subject for 24 hr and then detached. The condition of the skin after detachment was observed and evaluated according to the following evaluation criteria.

<Evaluation criteria>

⊙; no change from before adhesion
○; no change from before adhesion, but slight foreign sensation was felt on detachment
×; red flare and edema were observed after detachment, and irritation was felt on detachment
-; not evaluated

The evaluation results of the above are shown in Table 3.
Table 3

|  | shape retention | constant load (anchorage) | | | ball tackiness value - | | skin irritation |
|  |  | average (mm/h) | failure mode | evaluation | ball No. | evaluation |  |
|---|---|---|---|---|---|---|---|
| Example 1 | ○ | 229 | K | ○ | 18 | ⊙ | ○ |
| Example 2 | ○ | 277 | K | ○ | 18 | ⊙ | ○ |
| Example 3 | ○ | 235 | K | ○ | 16 | ○ | ○ |
| Example 4 | ○ | 225 | K | ○ | 16 | ○ | ○ |
| Example 5 | ○ | 175 | K | ⊙ | 17 | ⊙ | ⊙ |
| Example 6 | ○ | 152 | K | ⊙ | 20 | ⊙ | ⊙ |
| Example 7 | ○ | 74 | K | ⊙ | 20 | ⊙ | ⊙ |
| Comparative Example 1 | ○ | 2268 | K | × | 3 | × | - |
| Comparative Example 2 | ○ | 906 | T | × | 20 | ⊙ | - |

(continued)

| | shape retention | constant load (anchorage) | | | ball tackiness value - | | skin irritation |
|---|---|---|---|---|---|---|---|
| | | average (mm/h) | failure mode | evaluation | ball No. | evaluation | |
| Comparative Example 3 | × | thin layer sheet was not formed and measurement was not available | | | | | - |

As is clear from Table 3, each patch of Examples 1 - 7 showed good shape retention, good evaluation results of constant load (anchorage) and good ball tackiness value, whereby it was demonstrated that each patch does not fall off easily from the skin even when an external load is applied to the exposed surface of the support. In addition, problematic skin irritation was not observed.

In contrast, the patch of Comparative Example 1, which was prepared using a tetrafunctional crosslinking agent, showed shape retention but was evaluated to easily fall off from the skin due to an external load. The patch of Comparative Example 2, which was prepared using a tetrafunctional crosslinking agent and a catalyst, also showed shape retention but was cured immediately after drying. Thus, it showed an anchor failure in a constant load test, and failed to ensure a sufficient anchoring force to a support. Even if the anchoring force could have been ensured by hot pressing the patch of Comparative Example 2, the stress of 100% modulus measured as a flexibility index revealed that the followability to the skin became poor. Moreover, the patch of Comparative Example 3, which was prepared using a trifunctional crosslinking agent but without a catalyst, showed insufficient crosslinking formation and did not show shape retention, and therefore, a thin layer sheet-like adhesive layer could not be formed. The patches of Comparative Example 1 and Comparative Example 2 easily fell off from the skin, and a thin layer sheet-like adhesive layer was not formed in Comparative Example 3. Therefore, the patches of these Comparative Examples were not evaluated for the skin irritation.

[Examples 8, 9]

According to the formulation shown in Table 4, the patches of Examples 8 and 9 were prepared as in the above-mentioned Examples. The content (wt%) of each component to the total weight of the adhesive layer, the crosslinking agent used, the functional group ratio, and the kind of the catalyst are shown in Table 5.

[Table 4]

| | amount added (parts by weight) | | | | |
|---|---|---|---|---|---|
| | liquid rubber | tackifier | organic liquid component | crosslinking agent | catalyst |
| Example 8 | 100 | 100 | 50 | 1.74 | 0.10 |
| Example 9 | 100 | 100 | 50 | 1.74 | 1.20 |

[Table 5]

| | component | | | | | | | functional group ratio (%) |
|---|---|---|---|---|---|---|---|---|
| | liquid rubber (wt%) | tackifier (wt%) | organic liquid component (wt%) | crosslinking agent | | catalyst | | |
| | | | | (wt%) | kind | (wt%) | kind | |
| Example 8 | 39.70 | 39.70 | 19.85 | 0.69 | A | 0.04 | copper naphthenate | 30 |
| Example 9 | 39.53 | 39.53 | 19.77 | 0.69 | A | 0.47 | nickel naphthenate | 30 |

The patches of Examples 8 and 9 have similar properties as those of the above-mentioned patches of Examples 1 - 7.

INDUSTRIAL APPLICABILITY

The present invention provides a patch and a patch preparation less irritative to the skin, which resist falling off from the skin even when a load is applied to an exposed surface of a support of the patch or patch preparation and can be preferably used for the protection of an affected part on the skin and wounded surface, as well as transdermal absorption of a drug.

**Claims**

1. A patch comprising a support and an adhesive layer formed on at least one surface of the support, wherein the adhesive layer is obtainable by crosslinking a liquid rubber having a crosslinkable functional group in a molecule thereof in the presence of a crosslinking agent having a functional group number of 3 or below and a catalyst.

2. The patch according to claim 1, wherein the liquid rubber is a liquid isoprene rubber having a crosslinkable functional group in a molecule.

3. The patch according to claim 1 or 2, wherein the crosslinking is performed under conditions such that the proportion of the total number of the functional groups of the crosslinking agent to the total number of the functional groups of the liquid rubber is 30% - 100%.

4. The patch according to any one of claims 1 to 3, wherein the crosslinking is performed in the presence of an organic liquid component.

5. The patch according to claim 4, wherein the organic liquid component is one or more kinds selected from a fat, an oil, a hydrocarbon, a fatty acid ester and a higher fatty acid.

6. The patch according to claim 5, wherein the fatty acid ester is a fatty acid monoalkyl ester.

7. The patch according to claim 6, wherein the fatty acid monoalkyl ester is one or two kinds selected from isopropyl myristate and isopropyl palmitate.

8. The patch according to any one of claims 1 to 7, wherein the crosslinking is performed in the presence of a tackifier.

9. The patch according to any one of claims 1 to 8, wherein the adhesive layer has a thickness of not less than 40 $\mu$m.

10. The patch according to any one of claims 1 to 9, wherein the adhesive layer comprises a drug.

11. A method of making a patch as defined in any one of claims 1 to 10, which comprises forming on at least one surface of a support an adhesive layer by crosslinking a liquid rubber having a crosslinkable functional group in a molecule thereof in the presence of a crosslinking agent having a functional group number of 3 or below and a catalyst.

12. The method according to claim 11, comprising the steps of: providing the liquid rubber; adding the crosslinking agent and the catalyst thereto; applying the mixture to at least one surface of the support; and performing the crosslinking to form the adhesive layer.

13. The method according to claim 12, further comprising press-bonding a release liner to an adhesive face of the adhesive layer.

14. The method according to claim 11, comprising the steps of: providing the liquid rubber; adding the crosslinking agent and the catalyst thereto; applying the mixture on a release liner; performing the crosslinking to form the adhesive layer; and press-bonding the support to the adhesive layer.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 3220120 A